# EUROPÄISCHE PATENTANMELDUNG

(11) **EP 2 377 920 A1**
(43) Veröffentlichungstag der Anmeldung: **19.10.2011**
(21) Anmeldenummer: 10003967.6
(22) Anmeldetag: 15.04.2010
(51) Int. Cl.: C12N 1/20, C12Q 1/04

(54) **Verfahren zur unspezifischen Anreicherung von Mikroorganismen**

(71) Anmelder: Qiagen GmbH, 40724 Hilden (DE)
(72) Erfinder: Narz, Frank, Dr., 58456 Witten (DE)

(57) **Zusammenfassung**

Die vorliegende Erfindung betrifft ein Verfahren zur unspezifischen Anreicherung von Mikroorganismen aus komplexen Ausgangsmaterialien, wobei die die Mikroorganismen enthaltenden Ausgangsmaterialien in Kontakt gebracht werden mit Zellen des angeborenen Immunsystems, die Mikroorganismen an die Zellen des angeborenen Immunsystems gebunden werden und der Bindungskomplex vom komplexen Ausgangsmaterial abgetrennt wird.

SEQUENCE LISTING

<110> QIAGEN GmbH

<120> Verfahren zur unspezifischen Anreicherung von Mikroorganismen

<130> PA428-EP

<160> 3

<170> PatentIn version 3.3

<210> 1
<211> 23
<212> DNA
<213> Artificial Sequence

<220>
<223> DNA oligonucleotide with 6-FAM as fluorescence dye at its 5' end
and BHQ1 as quencher at its 3' end

<400> 1
ggaggcgatc tatgtcttgt cca 23

<210> 2
<211> 24
<212> DNA
<213> Artificial Sequence

<220>
<223> Oligonucleotide primer sequence

<400> 2
acatcttacc gcaactacga ccat 24

<210> 3
<211> 24
<212> DNA
<213> Artificial Sequence

<220>
<223> Oligonucleotide primer sequence

<400> 3
tagcatagtc tttgtcccac cgta 24

## Beschreibung

Die vorliegende Erfindung betrifft ein Verfahren zur unspezifischen Anreicherung von Mikroorganismen aus komplexen Ausgangsmaterialien, wobei die die Mikroorganismen enthaltenden Ausgangsmaterialien in Kontakt gebracht werden mit Zellen des angeborenen Immunsystems, die Mikroorganismen an die Zellen des angeborenen Immunsystems gebunden werden und der Bindungskomplex vom komplexen Ausgangsmaterial abgetrennt wird.

Mikroorganismen liegen in komplexen Ausgangsmaterialien wie beispielsweise Lebensmitteln oder Bodenproben im Allgemeinen in sehr geringer Konzentration vor. Um eine ausreichende Zahl an Mikroorganismen für deren Nachweis aus diesen Ausgangsmaterialien gewinnen zu können, müssen relativ hohe Mengen an Ausgangsmaterial verarbeitet werden. Da es in den meisten Verfahren nicht möglich ist, hohe Mengen an Ausgangsmaterial mit den darin enthaltenen Mikroorganismen zu prozessieren, müssen die Mikroorganismen zuvor aus diesen Ausgangsmaterialien angereichert werden. Erschwert wird diese Anreicherung dadurch, dass Ausgangsmaterialien wie Lebensmittel oder Bodenproben im Allgemeinen eine sehr komplexe Mischung darstellen, in denen viele verschiedene organische Moleküle aus verschiedenen Stoffklassen mit unterschiedlichen physikalischen und chemischen Eigenschaften vorhanden sind.

Aus diesem Grunde ist es schwierig, Mikroorganismen aus diesen Ausgangsmaterialien anzureichern, ohne weitere Bestandteile der Ausgangsmaterialien mit anzureichern, die die nachfolgende Behandlung der Mikroorganismen stören oder inhibieren könnten.

Aus dem Stand der Technik sind verschiedene Verfahren bekannt, mit deren Hilfe Mikroorganismen aus komplexen Ausgangsmaterialien angereichert werden können.

Zu den chemischen Verfahren zählt beispielsweise die Adsorption der Mikroorganismen an Ionenaustauscher-Matrices. Dadurch, dass die meisten Mikroorganismen bei pH-Werten von über 5 eine negative Ladung an der Oberfläche aufweisen, lassen sich viele Mikroorganismen an Anionenaustauscher-Matrices binden. Da aber auf der anderen Seite auch viele Substanzen, die in den komplexen Ausgangsmaterialien vorhanden sind, eine negative Ladung an der Oberfläche aufweisen, binden auch diese an die Anionenaustauscher-Matrix, so dass die Bindekapazität der Matrix für Mikroorganismen stark limitiert ist, so dass im Extremfall keine Mikroorganismen an die Matrix binden können, da alle Bindestellen bereits durch andere Bestandteile der Ausgangsmaterialien abgesättigt sind.

Ein weiteres chemisches Verfahren stellt die Bindung von Mikroorganismen an Lectine dar. Lectine sind Carbohydrat-bindende Proteine, die selektiv die Oberflächenkomponenten von Bakterien erkennen und binden. Dieses Verfahren hat den Nachteil, dass die Lectine bevorzugt an gram-positive Bakterien binden. Zwar werden auch gram-negative Bakterien mit allerdings geringerer Affinität gebunden, jedoch können andere Mikroorganismen wie beispielsweise Pilze oder Viren nicht von den Lectinen gebunden werden. Die Bindung von Mikroorganismen an Lectine ist folglich auf Bakterien, insbesondere auf gram-positive Bakterien beschränkt.

Ein weiteres chemisches Verfahren ist die Trennung der Mikroorganismen durch wässrige Zwei-Phase-Systeme. Bei diesem Verfahren werden die Mikroorganismen von den übrigen Bestandteilen des Ausgangsmaterials zwischen zwei nicht miteinander mischbaren flüssigen Phasen getrennt, die sich in ihrem Molekulargewicht voneinander unterscheiden. Dieses Verfahren hat den Nachteil, dass sich sehr viele Bestandteile des Ausgangsmaterials in derselben Phase anreichern wie die Mikroorganismen, sich die Mikroorganismen also nicht von allen Bestandteilen des Ausgangsmaterials abtrennen lassen. Zudem ist die Auftrennung in die einzelnen Phasen häufig unvollständig und führt zum Verlust eines Teils der Mikroorganismen in der zweiten Phase.

Zu den physikalischen Methoden, die zur Anreicherung von Mikroorganismen beschrieben sind, gehört beispielsweise die Zentrifugation. Wird mit relativ geringer g-Zahl zentrifugiert, so sedimentiert ein Großteil der Bestandteile des Ausgangsmaterials, während die Mikroorganismen im Überstand verbleiben. Dabei werden allerdings auch die Mikroorganismen, die mit schwereren Bestandteilen des Ausgangsmaterials assoziiert sind, sedimentiert und gehen somit verloren. Auf der anderen Seite lassen sich durch Zentrifugation bei geringer g-Zahl keine Bestandteile des Ausgangsmaterials von den Mikroorganismen abtrennen, die leichter als die Mikroorganismen sind, so dass nur eine grobe Anreicherung von Mikroorganismen durch dieses Verfahren erzielt werden kann.

Ein weiteres physikalisches Verfahren zur Anreicherung von Mikroorganismen ist die Filtration durch geeignete Filter. Die Filtration hat sich als ein relativ ungeeignetes Verfahren erwiesen, da größere Bestandteile des Ausgangsmaterials schnell den Filter verstopfen, so dass die Trennung sehr schnell zum Erliegen kommt. Des weiteren werden zusammen mit den Mikroorganismen auch viele Bestandteile des Ausgangsmaterials auflconzentriert, die den Nachweis der Mikroorganismen erschweren können.

Ein weiteres Verfahren zur Anreicherung von Mikroorganismen nutzt die Immunoaffinität von Mikroorganismen aus. Bei diesem Verfahren werden Antikörper an eine feste Phase immobilisiert, die spezifisch eine bestimmte Spezies Mikroorganismen binden können. Zwar ist dieses Verfahren sehr spezifisch, und keine weiteren Bestandteile des Ausgangsmaterials binden an die Antikörper, allerdings stellt dieses Verfahren kein generisches Verfahren dar, da nur eine bestimmte Spezies oder eine bestimmte Mikroorganismengruppe an die Antikörper binden kann, während andere in der Probe vorhandene Mikroorganismen nicht an die Antikörper binden können. Zudem ist dieses Verfahren durch die Notwendigkeit der Bereitstellung einer hohen Zahl von Antikörpern recht kostenintensiv.

Aufgabe der vorliegenden Erfindung ist es, die Nachteile der aus dem Stand der Technik bekannten Verfahren zu überwinden und ein generisches Verfahren zur Anreicherung von Mikroorganismen aus komplexen Ausgangsmaterialien zur Verfügung zu stellen, bei dem die Mikroorganismen nach der Abtrennung vom komplexen Ausgangsmaterial in einer höheren Konzentration vorliegen.

Diese Aufgabe wird gelöst durch ein Verfahren zur unspezifischen Anreicherung von Mikroorganismen aus komplexen Ausgangsmaterialien, aufweisend die folgenden Verfahrensschritte:
a) In Kontakt Bringen des Mikroorganismen enthaltenden komplexen Ausgangsmaterials mit Zellen des angeborenen Immunsystems;
b) Ausbilden eines Bindungskomplexes zwischen den Mikroorganismen und Zellen des angeborenen Immunsystems;
c) Abtrennung des Bindungskomplexes aus Schritt b) vom komplexen Ausgangsmaterial.

Das erfindungsgemäße Verfahren ist geeignet, unspezifisch Mikroorganismen aus den verschiedensten komplexen Ausgangsmaterialien anzureichern.

Dabei wird unter Anreicherung verstanden, dass die Mikroorganismen nach der Abtrennung des Bindungskomplexes vom komplexen Ausgangsmaterial in einer höheren Konzentration vorliegen als im komplexen Ausgangsmaterial, was beispielsweise durch eine Reduktion des Volumens, in dem die Mikroorganismen nach dem Verfahren vorliegen, erreicht werden kann.

Unter einem Mikroorganismus werden alle diejenigen Organismen verstanden, die vom angeborenen Immunsystem höherer Organismen als fremd erkannt werden. Dazu gehören beispielsweise neben gram-positiven und gram-negativen Bakterien sowie deren Dauerstadien auch Pilze und deren Dauerstadien, Viren sowie Archae-Bakterien sowie Bestandteile von diesen Organismen. Für das erfindungsgemäße Verfahren spielt es dabei keine Rolle, ob diese Mikroorganismen Stoffwechselaktivität aufweisen oder abgestorben sind oder ob es sich um Bestandteile dieser Mikroorganismen handelt, so lange diese vom angeborenen Immunsystem als fremd erkannt werden können.

In einer bevorzugten Ausführungsform handelt es sich bei den anzureichernden Mikroorganismen und gram-positive und/oder gram-negative Bakterien.

Als komplexes Ausgangsmaterial, aus dem die Mikroorganismen angereichert werden sollen, eignet sich prinzipiell jedes Ausgangsmaterial, in dem Mikroorganismen vorhanden sind oder sein können. Zu diesen Ausgangsmaterialien gehören beispielsweise Lebensmittel, die auf ihre Freiheit von Mikroorganismen hin überprüft werden sollen. Dazu gehören beispielsweise Fruchtsäfte, Milch, Fleisch, Obst, Wurstwaren, Käse und weitere Milcherzeugnisse. Aber auch andere komplexe Ausgangsmaterialien, aus denen Mikroorganismen nachgewiesen werden sollen, lassen sich im erfindungsgemäßen Verfahren als Ausgangsmaterialien verwenden. Dazu gehören beispielsweise Boden- oder Schlammproben oder auch Blut und andere Körperflüssigkeiten sowie Kosmetika.

Je nachdem, wie die komplexen Ausgangsmaterialien beschaffen sind, kann es erforderlich sein, die Ausgangsmaterialien einer Vorbehandlung zu unterziehen, bevor sie in Schritt a) des erfindungsgemäßen Verfahrens mit Zellen des angeborenen Immunsystems in Kontakt gebracht werden.

So ist es beispielsweise möglich, die Ausgangsmaterialien zunächst unter Bedingungen zu zentrifugieren, so dass die Mikroorganismen in Lösung bleiben, während Bestandteile der Ausgangsmaterialien unter diesen Bedingungen pelletieren. In diesem Fall wird mit dem Überstand mit den in Lösung vorhandenen Mikroorganismen weitergearbeitet.

Es ist aber auch möglich, dass die Mikroorganismen pelletieren, während Bestandteile der Ausgangsmaterialien unter diesen Bedingungen in Lösung bleiben. In diesem Fall wird mit dem Pellet mit den sich darin befindlichen Mikroorganismen weitergearbeitet.

Bei komplexen Ausgangsmaterialien, die überwiegend fest sind wie beispielsweise Bodenproben oder Fleisch kann es erforderlich sein, dass das Ausgangsmaterial zunächst zerkleinert, homogenisiert und/oder mit einer Flüssigkeit versetzt wird, bevor es in Schritt a) des erfindungsgemäßen Verfahrens mit Zellen des angeborenen Immunsystems in Kontakt gebracht wird. Möglichkeiten zur Zerkleinerung und/oder Homogenisierung der Ausgangsmaterialien sind dem Fachmann geläufig.

Auch wenn die Ausgangsmaterialien zunächst zerkleinert, homogenisiert und/oder mit einer Flüssigkeit versetzt worden sind, kann es erforderlich sein, dass die Ausgangsmaterialien noch weiter vorbehandelt werden müssen, beispielsweise durch Zentrifugation, bevor sie mit den Zellen des angeborenen Immunsystems in Kontakt gebracht werden.

Weitere Verfahren und Möglichkeiten, wie die komplexen Ausgangsmaterialien vorbehandelt werden können, sind dem Fachmann geläufig.

In Schritt a) des erfindungsgemäßen Verfahrens wird das komplexe Ausgangsmaterial, das die Mikroorganismen enthält, mit Zellen des angeborenen Immunsystems in Kontakt gebracht.

Die Zellen des angeborenen Immunsystems können dabei aus jedem beliebigen Organismus stammen, der ein angeborenes Immunsystem besitzt, das in der Lage ist, Mikroorganismen als dem Organismus fremd zu erkennen. Dabei sind nahezu alle mehrzelligen Organismen in der Lage, Mikroorganismen als fremd zu erkennen und weisen ein angeborenes Immunsystem auf. Zu diesen Organismen, die Zellen eines angeborenen Immunsystems aufweisen, gehören beispielsweise der Mensch, weitere Säugetiere sowie weitere Wirbeltiere, Pflanzen, Insekten sowie weitere wirbellose Tiere.

In einer bevorzugten Ausführungsform stammen die Zellen des angeborenen Immunsystems aus Säugetieren.

In einer besonders bevorzugten Ausführungsform sind die Zellen des angeborenen Immunsystems menschlichen Ursprungs.

Die Zellen des angeborenen Immunsystems können dabei als einzelne Zellen isoliert vorliegen, sie können aber auch einen Zell- oder Gewebeverband aus Zellen des angeborenen Immunsystems bilden. Desweiteren ist es möglich, dass die Zellen des angeborenen Immunsystems sich in Zell- oder Gewebeverbänden befinden, in denen weitere Zellen vorkommen, die nicht Bestandteil des angeborenen Immunsystems sind.

In einer Ausführungsform sind die Zellen des angeborenen Immunsystems primäre Zellen, die einem Organismus als Einzelzelle, als Zell- oder als Gewebeverband entnommen worden sind. Diese primären Zellen können direkt in das erfindungsgemäße Verfahren eingesetzt werden, sie können aber auch vor ihrem Einsatz im erfindungsgemäßen Verfahren noch in vitro kultiviert worden sein.

In einer bevorzugten Ausführungsform sind die primären Zellen des angeborenen Immunsystems myeloide Zellen. Zu diesen zählen beispielsweise Monocyten, Makrophagen, neutrophile, eosinophile und basophile Granulozyten sowie natürliche Killerzellen. Diese können als Mischung einzelner Zelltypen eingesetzt werden, es kann aber auch nur ein Zelltypus eingesetzt werden.

In einer besonders bevorzugten Ausführungsform handelt es sich bei den myeloiden Zellen um Makrophagen.

In einer weiteren Ausführungsform sind die Zellen des angeborenen Immunsystems immortalisierte Zellen einer oder mehrerer entsprechender Zelllinien, die in Zellkultur gehalten werden.

In einer bevorzugten Ausführungsform sind diese immortalisierten Zellen aus dem Blutsystem abgeleitet, wie zum Beispiel Leukämie-, Lymphoma- oder Myeloma-Zellen.

In einer besonders bevorzugten Ausführungsform handelt es sich bei den immortalisierten Zellen um Zellen der Zelllinie THP1, U937, K562 oder einer Mischung davon.

Weitere geeignete Zelllinien sind dem Fachmann geläufig.

In Schritt b) des erfindungsgemäßen Verfahrens binden die Mikroorganismen an die Zellen des angeborenen Immunsystems und bilden so einen Bindungskomplex aus. Im Sinne der vorliegenden Erfindung wird unter Bindung jegliche Wechselwirkung zwischen dem Mikroorganismus und der Zelle des angeborenen Immunsystems verstanden. Die Zellen des angeborenen Immunsystems sind in der Lage, körperfremde Zellen, Zellbestandteile und Organismen auf molekularer Ebene zu erkennen und mit diesen in Wechselwirkung zu treten. Dabei erkennen die Zellen des angeborenen Immunsystems nicht spezifisch einen bestimmten körperfremden Mikroorganismus oder einen Teil davon, wie dies beispielsweise bei Antikörper-Antigen-Wechselwirkungen der Fall ist. Vielmehr ist diese Wechselwirkung unspezifisch, so dass die Zellen des angeborenen Immunsystems ein breites Spektrum an verschiedenen Mikroorganismen oder Teile dieser Mikroorganismen auf molekularer Ebene erkennen können.

Die Wechselwirkung zwischen Zelle und Mikroorganismus kann beispielsweise aus einer Bindung zwischen Zelle und Mikroorganismus im engeren Sinne bestehen, die auf einer molekularen Wechselwirkung zwischen diesen beiden Partnern beruht, die Wechselwirkung kann aber auch darin bestehen, dass die Zelle des angeborenen Immunsystems den Mikroorganismus phagocytiert oder ihn auf andere Art in sein Zellinneres inkorporiert. Weitere Möglichkeiten der Wechselwirkung zwischen Zelle und Mikroorganismus sind dem Fachmann geläufig.

In einer Ausführungsform weisen die Zellen des angeborenen Immunsystems Stoffwechselaktivität auf. Dabei sind die Zellen in der Lage, einige oder die meisten ihrer Stoffwechselwege und/oder biologischen Prozesse durchzuführen. Dazu gehören beispielsweise die Wechselwirkung mit anderen Zellen, Zellverbänden oder Geweben oder auch die Phagocytose von als körperfremd erkannten Substanzen und Mikroorganismen. Sofern die Zellen aufgrund ihres Differenzierungsgrades dazu in der Lage sind, gehört dazu auch die Fähigkeit zur Zellteilung.

In einer weiteren Ausführungsform weisen die Zellen des angeborenen Immunsystems keine Stoffwechselaktivität mehr auf. Diese Zellen können beispielsweise fixiert sein und sind dadurch vor Autolyse geschützt, so dass ihre Zellstrukturen weitgehend erhalten bleiben. Zur Fixierung von Zellen bietet sich beispielsweise die Quervernetzung von Zellbestandteilen durch Aldehyde wie Formaldehyd oder Glutaraldehyd an. Ein weiteres Verfahren zur Fixierung ist beispielsweise die Fixierung durch Wasserentzug, wie sie durch Chemikalien wie Aceton oder Alkohole wie beispielsweise Ethanol oder Methanol erreicht werden kann. Weitere Verfahren zur Fixierung von Zellen, so dass die Zellstrukturen weitgehend erhalten bleiben, sind dem Fachmann geläufig.

Unabhängig davon, ob die Zellen noch Stoffwechselaktivität aufweisen oder nicht, können diese an eine feste Phase immobilisiert sein. Die Immobilisierung kann dabei über kovalente Bindung oder über andere Mechanismen wie Van-Der-Waals-Kräfte, Bindung über funktionelle Gruppen, Rezeptor-Ligand-Bindung, Antikörper-Antigen-Bindung oder elektrostatische Interkation erfolgen. Weitere Möglichkeiten der Immobilisierung von Zellen an feste Phasen sind dem Fachmann geläufig.

Als feste Phasen eignen sich prinzipiell alle möglichen Formen und Materialien. Beispiele für feste Phasen sind planare, konvexe oder konkave Oberflächen, magnetische und nicht magnetische Partikel, Beschichtungen von Reagiergefäßen, Mikrotiterplatten, Objektträger und viele weitere mehr. Dabei können die festen Phasen aus beliebigem Material hergestellt sein, solange es möglich ist, direkt oder indirekt an diese festen Phasen Zellen des angeborenen Immunsystems zu immobilisieren. Dem Fachmann sind viele weitere geeignete feste Phasen geläufig, die für das erfindungsgemäße Verfahren verwendet werden können.

Unabhängig davon, ob die Zellen des angeborenen Immunsystems an eine feste Phase immobilisiert sind oder nicht, weisen diese Zellen in einer besonderen Ausführungsform intrazellulär magnetische Partikel auf. Die magnetischen Partikel können dabei ferro-, ferri-oder superparamagnetische Partikel sein. Dem Fachmann sind viele unterschiedliche Verfahren geläufig, wie solche magnetischen Partikel in das Zellinnere gelangen können. Möglichkeiten sind beispielsweise das Inkorporieren durch Phagocytose, das Hineinschleusen mittels unterschiedlicher Transfektionsmethoden oder das Beschießen der Zellen mit Hilfe einer Partikelkanone.

In Schritt c) des erfindungsgemäßen Verfahrens wird der Bindungskomplex aus Schritt b) vom komplexen Ausgangsmaterial abgetrennt.

Möglichkeiten, den Bindungskomplex vom komplexen Ausgangsmaterial zu trennen, sind aus dem Stand der Technik in ausreichendem Maße bekannt. Weist der Bindungkomplex eine andere Dichte auf als das komplexe Ausgangsmaterial, lässt sich der Bindungskomplex einfach durch Zentrifugation vom komplexen Ausgangsmaterial abtrennen. Eine weitere Möglichkeit zur Abtrennung stellt die Filtration dar, wenn der Bindungskomplex eine andere Größe als das komplexe Ausgangsmaterial aufweist. Eine weitere Möglichkeit zur Abtrennung besteht in der Hilfe mit Antikörpern, die gegen ein Epitop auf der Oberfläche der Zellen des angeborenen Immunsystems gerichtet sind.

Das Abtrennen vom komplexen Ausgangsmaterial wird erleichtert, wenn die Zellen des angeborenen Immunsystems an eine feste Phase gekoppelt sind, da sich die feste Phase mit dem nun daran befindlichen Komplex aus Zellen des angeborenen Immunsystems mit den Mikroorganismen leicht von den übrigen Bestandteilen des komplexen Ausgangsmaterials abtrennen lässt. Das Abtrennen kann beispielsweise durch einfaches Entnehmen der festen Phase aus dem Bindungsansatz mit dem komplexen Ausgangsmaterial erfolgen oder auch beispielsweise durch Dekantieren des Bindungsansatzes, wobei der Bindungskomplex aus Zellen des angeborenen Immunsystems mit den daran gebundenen Mikroorganismen im Reagiergefäß zurückgehalten wird.

Wenn die feste Phase magnetisch ist, oder wenn die Zellen des angeborenen Immunsystems magnetische Partikel inkorporiert haben, lässt sich die Trennung des Bindungskomplexes von den übrigen Bestandteilen des Bindungsansatzes mit dem komplexen Ausgangsmaterial einfach durch magnetische Separation erreichen.

Weitere Möglichkeiten, den Bindungskomplex vom Bindungsansatz zu trennen, sind dem Fachmann geläufig.

Nachdem in Schritt c) der Bindungskomplex vom komplexen Ausgangsmaterial abgetrennt worden ist, kann der Bindungskomplex aus Zellen des angeborenen Immunsystems mit Mikroorganismen gegebenenfalls gewaschen werden, um noch eventuell vorhandene Reste des komplexen Ausgangsmaterials vom Bindungskomplex zu entfernen.

Nachdem durch das erfindungsgemäße Verfahren die Mikroorganismen unspezifisch angereichert worden sind, können diese Mikroorganismen nun spezifisch mit dem Fachmann geläufigen Verfahren nachgewiesen und/oder quantifiziert werden.

Beispielsweise lassen sich die Mikroorganismen mit Hilfe bestimmter Proteine an ihrer Oberfläche nachweisen. Der Nachweis dieser Proteine kann dann beispielsweise über Immunfluoreszenz, Western-Blot, FACS, Durchflusszytometrie und weitere dem Fachmann bekannte Proteinnachweisverfahren erfolgen.

Die Mikroorganismen lassen sich aber auch mit Hilfe ihrer Nukleinsäuren nachweisen und quantifizieren. Möglichkeiten dafür sind beispielsweise Nukleinsäure-Hybridisierungstechniken, zu denen beispielsweise Northem-Blots, Southern-Blots, Microarrays und Dot-Blots gehören.

Eine weitere Möglichkeit, Mikroorganismen spezifisch über ihre Nukleinsäuresequenz nachzuweisen, besteht in Amplifikationstechniken wie PCR mit ihren Variationsmöglichkeiten wie beispielsweise Endpunkt-PCR oder Real-Time PCR sowie in isothermalen Amplifikationsverfahren wie beispielsweise Rolling Circle Amplification (RCA) mit ihren Variationsmöglichkeiten.

Dem Fachmann sind weitere Möglichkeiten zum Nachweis von Mikroorganismen über ihre Nukleinsäuren geläufig.

Abbildungen:

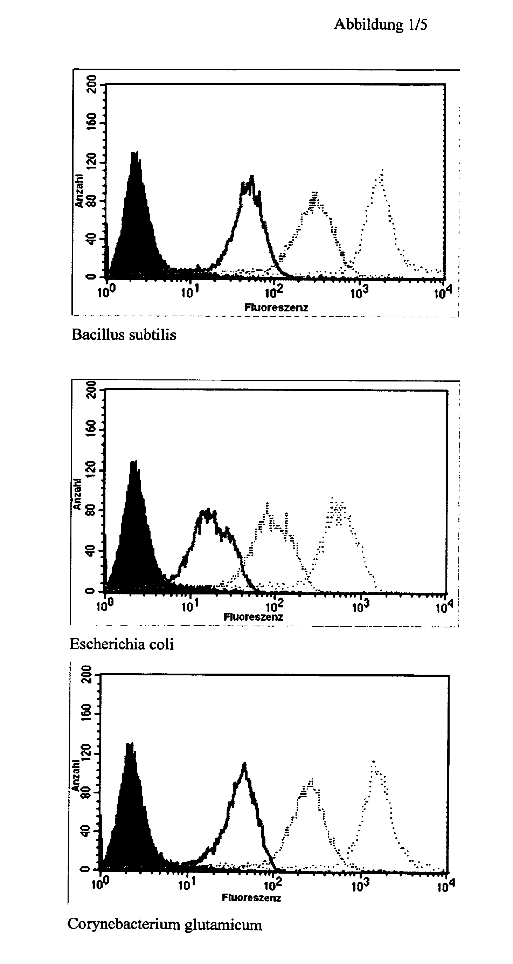

Abbildung 1: Analyse der Bindung verschiedener Fluoreszenz-markierter Mikroorgansimen in verschiedener Anzahl an THP1 Zellen mittels Durchflusszytometrie. Die gefüllte Fläche repräsentiert die Nullkontrolle, die durchgezogene Linie 10⁶ Mikroorganismen, die gestrichelte Linie 10⁷ Mikroorganismen, die gepunktete Linie 10⁸ Mikroorganismen.

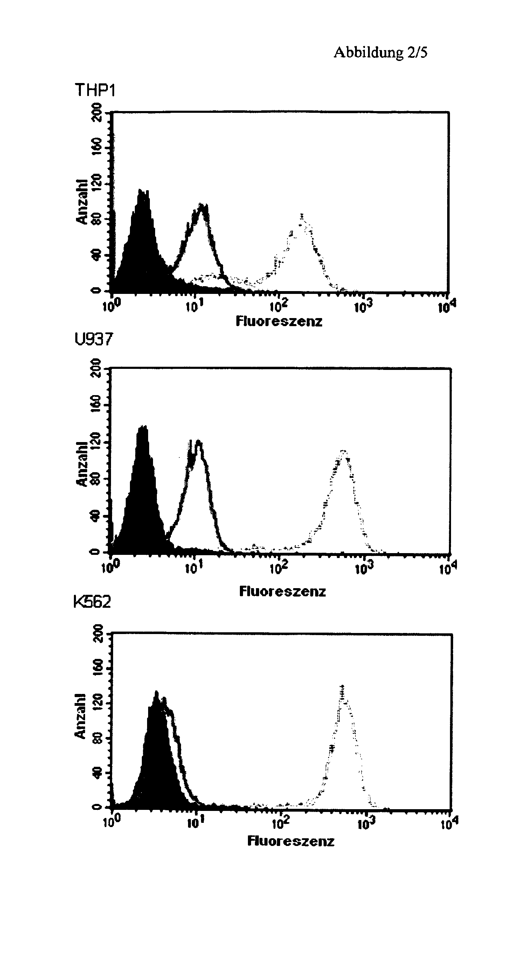

Abbildung 2: Analyse der Bindung von Fluoreszenz-markierten Bacillus subtilis in verschiedener Anzahl an THP1, U937 bzw. K562 Zellen mittels Durchflusszytometrie. Die gefüllte Fläche repräsentiert die Nullkontrolle, die durchgezogene Linie 10⁵ Mikroorganismen, die gestrichelte Linie 10⁷ Mikroorganismen.

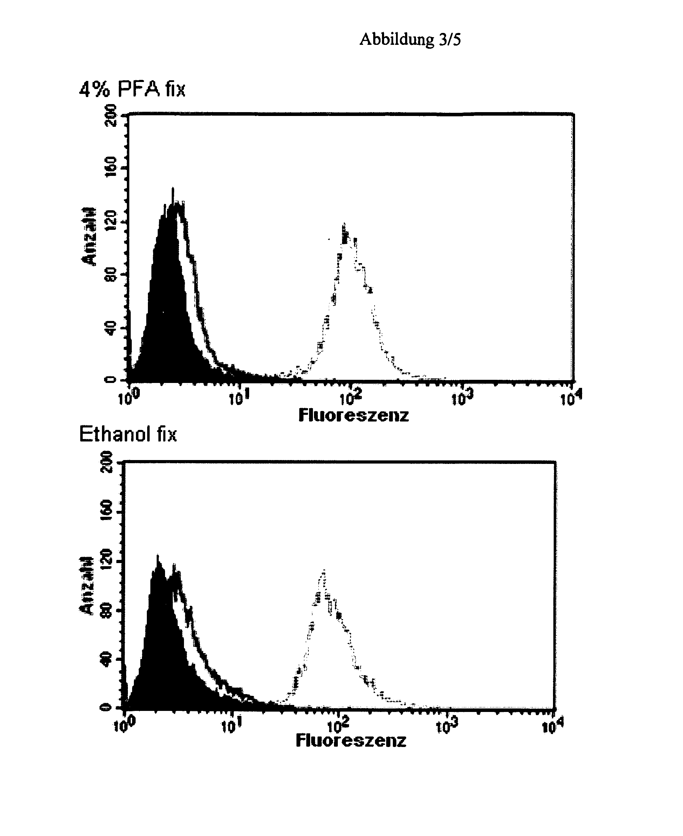

Abbildung 3: Analyse der Bindung von Fluoreszenz-markierten Bacillus subtilis in verschiedener Anzahl an fixierte THP1 Zellen mittels Durchflusszytometrie. Die gefüllte Fläche repräsentiert die Nullkontrolle, die durchgezogene Linie 10⁵ Mikroorganismen, die gestrichelte Linie 10⁷ Mikroorganismen.

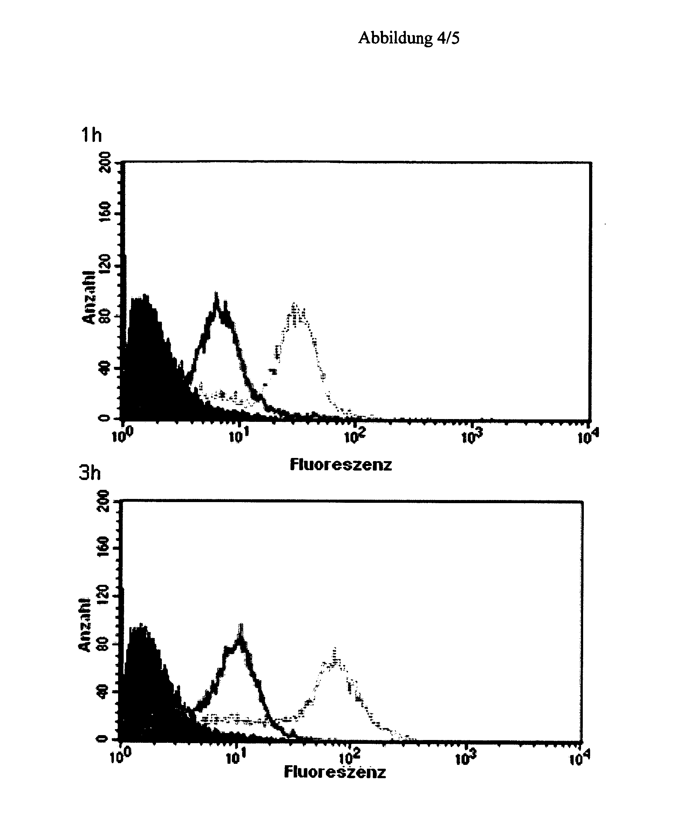

Abbildung 4: Analyse der Bindung von Fluoreszenz-markierten Bacillus subtilis in verschiedener Anzahl an THP1 Zellen in Milch zu verschiedenen Zeitpunkten mittels Durchflusszytometrie. Die gefüllte Fläche repräsentiert die Nullkontrolle, die durchgezogene Linie 10⁶ Mikroorganismen, die gestrichelte Linie 10⁸ Mikroorganismen.

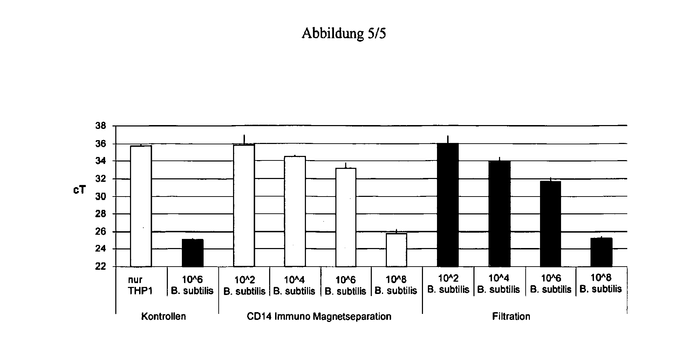

Abbildung 5: Nachweis der an THP1 gebundenen Bakterien über RT-PCR. Auf der X-Achse sind die unterschiedliche Anzahl der eingesetzten Bakterien sowie die Art der Abtrennung des Bindungskomplexes vom Ausgangsmaterial angegeben. Auf der Y-Achse ist der cT-Wert angegeben.

Beispiele:
Die nachfolgenden Beispiele sind dazu gedacht, die Erfindung weiter zu erläutern, ohne dass diese auf die Ausführungsbeispiele beschränkt sein soll.

Folgende Oligonukleotide oder Primer-/Probesequenzen fanden Verwendung:
BacSub-Probe: 5 '-6-FAM-GGAGGCGATCTATGTCTTGTCCA-BHQ 1
BacSub-FWD: 5 '-ACATCTTACCGCAACTACGACCAT
BacSub-REV: 5'-TAGCATAGTCTTTGTCCCACCGTA

### Beispiel 1:

Bindung verschiedener Bakterien an THP1 Zellen

Jeweils 10⁹ Escherichia coli (gram-negatives Bakterium), Bacillus subtilis und Corynebacterium glutamicum (beide gram-positive Bakterien) wurden in 1 ml PBS gegeben und mit dem Fluoreszenzfarbstoff SYTO BC des Bacteria Counting Kits (Invitrogen, Carlsbad, USA) für 10 min bei Raumtemperatur in der Dunkelheit unter Rotation angefärbt. Anschließend wurden die Bakterien für 5 min bei 14.000 U/min abzentrifugiert und mit 1 ml PBS gewaschen. 10⁶, 10⁷ bzw. 10⁸ dieser Fluoreszenz-markierten Bakterien wurden jeweils zu 2x 10⁵ Zellen der menschlichen Monocyten-Zelllinie THP1 gegeben und für 30 min bei 37 °C unter Schütteln inkubiert. Nach einer Zentrifugation von 5 min bei 1.000 U/min wurde der Bindungskomplex mit PBS gewaschen und nach erneuter Zentrifugation im Durchflusszytometer analysiert.

Wie in Abbildung 1 gezeigt, wurden die drei unterschiedlichen Mikroorganismen in gleichem Maße bei den unterschiedlichen verwendeten Mikroorganismen-Mengen an die THP1 Zellen gebunden, was verdeutlicht, dass die Wechselwirkung zwischen den Zellen des angeborenen Immunsystems mit den Mikroorganismen eine generische Wechselwirkung ist und nicht spezifisch auf eine Art oder Gruppe von Mikroorganismen beschränkt ist.

### Beispiel 2:

Bindung von Bacillus subtilis an unterschiedliche Zelllinien

Vorgegangen wurde wie in Beispiel 1 beschrieben, nur dass diesmal als Mikroorganismus nur Bacillus subtilis verwendet wurde und dieser in Mengen von 10⁵ und 10⁷ Bakterien eingesetzt wurde. Im Unterschied zu Beispiel 1 wurden in Beispiel 2 als Zellen des angeborenen Immunsystems zusätzlich zur monocytischen Zelllinie THP 1 die ebenfalls humane monocytische Zelllinie U937 sowie die humane B-Zelllinie K562 verwendet. Abbildung 2 zeigt, dass THP1 und U937 Zellen in etwa mit gleicher Effizienz Bacillus subtilis banden. Im Gegensatz dazu war die Effizienz bei K562 Zellen deutlich geringer. Bei einer Menge von 10⁵ B. subtilis (ununterbrochene Linie) zeigte sich kaum ein Unterschied im FACS im Vergleich zur Kontrolle ohne eingesetzte B. subtilis (gefüllte Fläche). Wurden hingegen 10⁷ B. subtilis eingesetzt, so zeigte sich für die Zelllinie K562 in etwa dieselbe Effizienz wie für THP1 und U937. Dieses Experiment verdeutlicht, dass nicht nur Zellen der Zelllinie THP1 sondern auch weitere Zelltypen des angeborenen Immunsystems in der Lage sind, Mikroorganismen unspezifisch zu binden.

### Beispiel 3:

Bindung von Bacillus subtilis an fixierte THP1 Zellen

Wurden in den Beispielen 1 und 2 THP1 Zellen verwendet, die Stoffwechselaktivität aufwiesen, so wurden in Beispiel 3 stattdessen THP1 Zellen verwendet, die fixiert worden waren. Zur Fixierung wurden zwei unterschiedliche Verfahren verwendet. Im ersten Fixierungsansatz wurden 2x 10⁵ THP1 Zellen für 10 min bei Raumtemperatur in 4% Paraformaldehyd (PFA) inkubiert, im zweiten Fixierungsansatz für 10 min bei Raumtemperatur in 100% Ethanol. Während Paraformaldehyd die Amino-Reste in Proteinen verknüpft, bewirkt Ethanol eine schnelle Dehydrierung der Zellen. Die weiteren Versuche wurden durchgeführt wie in Beispiel 2 beschrieben, als Mikroorganismus wurde Bacillus subtilis eingesetzt.

Abbildung 3 zeigt, dass Mikroorganismen nicht nur von Zellen des angeborenen Immunsystems, die Stoffwechselaktivität aufweisen, gebunden werden können, sondern auch von fixierten Zellen. Zwar war der Nachweis von 10⁵ Mikroorganismen in diesem Experiment weniger effizient als der Nachweis mit Zellen, die noch Stoffwechselaktivität aufweisen, jedoch lassen sich auch mit fixierten Zellen 10⁵ Mikroorganismen im FACS sicher nachweisen, nachdem sie aus dem Ausgangsmaterial mit fixierten Zellen des angeborenen Immunsystems angereichert worden waren.

### Beispiel 4:

Anreicherung von Mikroorganismen aus einem komplexen Ausgangsmaterial

Vorgegangen wurde im Wesentlichen wie in Beispiel 1 beschrieben, nur dass in diesem Experiment 10⁶ und 10⁸ Bacillus subtilis in die Experimente eingesetzt wurden und diese aus einem komplexen Ausgangsmaterial, Vollmilch mit 1,5% Fettanteil, aufgereinigt wurden. Dazu wurden die Mikroorganismen in 25 ml Vollmilch gegeben und anschließend für 5 min zentrifugiert, um das unlösliche Material inklusive der Bakterien zu pelletieren. Anschließend wurde das Pellet in 25 ml Phosphate Buffered Saline (PBS) aufgenommen und 2x 10⁵ THP1 Zellen hinzugefügt. Inkubiert wurde für 1 bzw. 3 Stunden bei Raumtemperatur unter Rotation. Abbildung 4 zeigt, dass sich die Bakterien mit dem erfindungsgemäßen Verfahren auch anreichern und nachweisen lassen, wenn diese in einem komplexen Ausgangsmaterial wie Milch in relativ geringer Konzentration vorliegen. Dabei waren die Ergebnisse vergleichbar, wenn die Mikroorganismen für 1 bzw. für 3 h mit den Zellen des angeborenen Immunsystems in Kontakt gebracht worden waren.

### Beispiel 5:

Anreicherung von Bacillus subtilis mit Nachweis über RT-PCR 10², 10⁴, 10⁶ bzw. 10⁸ Bacillus subtilis wurden mit 2x 10⁵ THP1 Zellen in Zellkulturmedium (RPMI (Invitrogen) mit 10% Fötalem Kalbserum (PAA, Pasching, Österreich) inkubiert. Nicht gebundene Mikroorganismen wurden aus dem Bindungskomplex entweder durch Filtration durch einen 2 µm PCTE Filter (Sterlitech, Kent, USA) oder durch immunomagnetische Separation mit Hilfe eines biotinylierten CD 14 Antikörpers und BiotinBinder Magnetpartikel (Invitrogen) entfernt. Die genomische DNA aus dem Bindungskomplex wurde mit Hilfe des QIAamp Kits für bakterielle gDNA (QIAGEN, Hilden, Deutschland) isoliert. Die isolierte gDNA enthielt das Erbmaterial sowohl der THP1 Zellen als auch von B. subtilis.

In die nachfolgende RT-PCR wurden 5 µ1 dieser Eluate eingesetzt, in der ein Teilbereich der gDNA von B. subitilis amplifiziert wurde. Das Gesamtvolumen der RT-PCR betrug 20 µ1 mit folgenden weiteren Bestandteilen:
10 µ1 Quantitect Multiplex PCR Mix (QIAGEN)
0,4 µM BacSub-FWD Primer
0,4 µM BacSub-REV Primer
0,2 µM BacSub-Probe

Aufgefüllt wurde der Reaktionsansatz mit Wasser.

Die RT-PCR wurde im 384-Well-Format in einem ABI 7900HT durchgeführt, wobei folgendes Programm durchlaufen wurde:
1)15 min 95 °C
2) 40 Zyklen von:
   15 s 94°C
   30 s 55 °C
   30 s 72 °C

Als Kontrolle wurden 10⁶B. subtilis (100% Kontrolle) bzw. 2x 10⁵ THP1 (0% Kontrolle) eingesetzt.

Abbildung 5 zeigt, dass sich die Mikroorganismen nach ihrer Anreicherung mit dem erfmdungsgemäßen Verfahren auch mittels einer RT-PCR nachweisen und quantifizieren lassen. Dabei lassen sich bereits 10.000 Mikroorganismen, die aus dem Ausgangsmaterial angereichert wurden, nachweisen.

## Patentansprüche

1. Verfahren zur unspezifischen Anreicherung von Mikroorganismen aus komplexen Ausgangsmaterialien, aufweisend die folgenden Verfahrensschritte:
a) In Kontakt Bringen des Mikroorganismen enthaltenden komplexen Ausgangsmaterials mit Zellen des angeborenen Immunsystems;
b) Ausbilden eines Bindungskomplexes zwischen den Mikroorganismen und Zellen des angeborenen Immunsystems;
c) Abtrennung des Bindungskomplexes aus Schritt b) vom komplexen Ausgangsmaterial.

2. Verfahren nach Anspruch 1, **dadurch gekennzeichnet, dass** die Zellen des angeborenen Immunsystems aus Säugetieren stammen.

3. Verfahren nach Anspruch 2, **dadurch gekennzeichnet, dass** die Zellen des angeborenen Immunsystems menschlichen Ursprungs sind.

4. Verfahren nach einem der Ansprüche 1 bis 3, **dadurch gekennzeichnet, dass** die Zellen primäre Zellen sind.

5. Verfahren nach Anspruch 4, **dadurch gekennzeichnet, dass** die primären Zellen myeloide Zellen sind.

6. Verfahren nach Anspruch 5, **dadurch gekennzeichnet, dass** die myeloiden Zellen Makrophagen sind.

7. Verfahren nach einem der Ansprüche 1 bis 3, **dadurch gekennzeichnet, dass** die Zellen immortalisierte Zellen einer Zelllinie sind.

8. Verfahren nach Anspruch 7, **dadurch gekennzeichnet, dass** die immortalisierten Zellen einer Zelllinie abgeleitet sind aus dem Blutsystem.

9. Verfahren nach Anspruch 7, **dadurch gekennzeichnet, dass** die immortalisierten Zellen einer Zelllinie THP1, U937 oder K562 oder eine Mischung davon sind.

10. Verfahren nach einem der Ansprüche 1 bis 9, **dadurch gekennzeichnet, dass** die Zellen des angeborenen Immunsystems Stoffwechselaktivität aufweisen.

11. Verfahren nach einem der Ansprüche 1 bis 10, **dadurch gekennzeichnet, dass** die Zellen des angeborenen Immunsystems an einer festen Phase immobilisiert sind.

12. Verfahren nach einem der Ansprüche 1 bis 11, **dadurch gekennzeichnet, dass** die Zellen des angeborenen Immunsystems intrazellulär magnetische Partikel aufweisen.
